# EUROPÄISCHE PATENTSCHRIFT

(11) **EP 1 467 960 B1**
(45) Veröffentlichungstag und Bekanntmachung des Hinweises auf die Patenterteilung: **14.02.2007**
(21) Anmeldenummer: 03729477.4
(22) Anmeldetag: 16.01.2003
(51) Int. Cl.: C07C 45/50, C07C 29/16, C07C 47/02, C07C 31/02

(54) **VERFAHREN ZUR HYDROFORMYLIERUNG VON OLEFINEN MIT 2 BIS 6 KOHLENSTOFFATOMEN**
METHOD FOR THE HYDROFORMYLATION OF OLEFINS WITH 2 TO 6 CARBON ATOMS
PROCEDE D'HYDROFORMYLATION D'OLEFINES COMPORTANT 2 A 6 ATOMES DE CARBONE

(30) Priorität: 17.01.2002 DE 10201676
(43) Veröffentlichungstag der Anmeldung: 20.10.2004
(73) Patentinhaber: BASF Aktiengesellschaft, 67056 Ludwigshafen (DE)
(72) Erfinder: RICHTER, Wolfgang, 67157 Wachenheim (DE); MÜLLER, Rolf, 67125 Dannstadt-Schauernheim (DE); KROKOSZINSKI, Roland, 67273 Weisenheim am Berg (DE)
(74) Vertreter: Reitstötter - Kinzebach
(86) Internationale Anmeldenummer: PCT/EP2003/000419
(87) Internationale Veröffentlichungsnummer: WO 2003/059859

(56) Entgegenhaltungen:
- WO-A-97/20793
- DE-A- 10 031 519
- DE-A- 19 836 807
- "Ullmann's Encyclopedia of Industrial Chemistry, 5th edition, vol. B4" 1992 , VCH VERLAGSGESELLSCHAFT , WEINHEIM (DE) XP002238440 Seiten 172-174, Abbildungen 5, 6 and 9;

## Beschreibung

Die vorliegende Erfindung betrifft ein Verfahren zur Hydroformylierung, bei dem man wenigstens ein Olefin mit 2 bis 6 Kohlenstoffatomen mit Kohlenmonoxid und Wasserstoff in Gegenwart eines Hydroformylierungskatalysators in einer Reaktionszone kontinuierlich umsetzt.

Die Hydroformylierung oder Oxo-Synthese ist ein wichtiges großtechnisches Verfahren zur Herstellung von Aldehyden aus Olefinen, Kohlenmonoxid und Wasserstoff. Diese Aldehyde können gegebenenfalls im gleichen Arbeitsgang oder nachfolgend in einem getrennten Hydrierschritt mit Wasserstoff zu den entsprechenden Alkoholen hydriert werden. Die Hydroformylierung erfolgt in Anwesenheit von homogen im Reaktionsmedium gelösten Katalysatoren. Als Katalysatoren werden dabei im Allgemeinen Verbindungen oder Komplexe von Metallen der VIII. Nebengruppe, speziell Co-, Rh-, Ir-, Pd-, Pt- oder Ru-Verbindungen bzw. -komplexe eingesetzt, die unmodifiziert oder z. B. mit amin- oder phosphinhaltigen Verbindungen modifiziert sein können.

Die Hydroformylierung ist eine exotherme Reaktion, bei der insbesondere beim Einsatz kurzkettiger Olefine eine beträchtliche Wärmemenge freigesetzt wird, die aus dem zur Umsetzung eingesetzten Reaktor abgeführt werden muss. Es ist bekannt, zur Kühlung des Reaktorinhalts Wärmetauscher, z. B. in Form von Kühlschlangen, einzusetzen, die beispielsweise außen auf dem Reaktormantel aufgeschweißt sind oder durch das Innere des Reaktors geführt werden und die üblicherweise von einem Hilfsmedium zur Wärmeübertragung (Kühlmittel) durchflossen werden. Bei diesen Hilfsmedien handelt es sich beispielsweise um Wasser oder um andere Flüssigkeiten, die eine hohe Wärmeleitfähigkeit und -kapazität aufweisen. Mit dem Hydroformylierungsreaktor verbundene Wärmeaustauscher weisen jedoch mehrere Nachteile auf. So sind Reaktoren mit auf dem Reaktormantel aufgeschweißten Kühlschlangen in ihrer Größe begrenzt, da mit zunehmender Reaktorgröße die zur Kühlung zur Verfügung stehende Oberfläche im Vergleich zum Reaktorvolumen zu gering wird. Reaktoren mit innenliegenden Wärmetauschern, wie z. B.

Kühlregistern, sind baulich aufwendig und kostspielig. Zudem besteht bei Reaktoren mit außen- und/oder innenliegenden Wärmeaustauschern vielfach die Notwendigkeit, den Reaktorinhalt durch geeignete Maßnahmen, wie den Einsatz von Rührern, zu durchmischen, um eine effektive Abfuhr der Reaktionswärme zu gewährleisten.

Die deutschen Patentanmeldungen DE-A-100 31 517, 100 31 518, 100 31 519 und 100 31 520 beschreiben Verfahren zur Hydroformylierung von Olefinen.

Die US 4,577,043 beschreibt ein Hydroformylierungsverfahren zur Herstellung von Aldehyden in Gegenwart von Wasser und eines wasserlöslichen Katalysators, bei dem man aus dem Reaktor einen produkthaltigen Strom austrägt, diesen zunächst in eine flüssige und eine gasförmige Phase und die flüssige Phase weiter in eine wässrige und eine organische Phase auftrennt, wobei die einzelnen Trennschritte ohne vorherige Kühlung des Stroms erfolgen. Erst nach der Auftrennung wird die organische Phase abgekühlt, wobei ein Wärmeaustauscher eingesetzt werden kann, der zum Erwärmen des in der Reaktion eingesetzten Synthesegases und/oder des Olefins dient.

Die US 4,523,036 beschreibt ein kontinuierliches Verfahren zur Herstellung von Aldehyden aus Olefinen und Synthesegas, bei dem man zur Gewinnung der Reaktionsprodukte eine Phasentrennung ohne vorherige Kühlung durchführt. Diese Phasentrennung kann nach einer ersten Ausführungsform in einem von dem Hydroformylierungsreaktor räumlich getrennten Phasentrenngefäß erfolgen. Anschließend kann in einem nachgeschalteten Wärmeaustauscher die Prozessabwärme genutzt werden. Nach einer zweiten Ausführungsform erfolgt die Phasentrennung in speziellen Aufbauten am Kopf des Reaktors. Dabei kann die Prozessabwärme mit einem Kühlregister abgeführt werden. In beiden Fällen kann die Reaktionswärme ohne den Einsatz eines Hilfsmediums zur Wärmeübertragung abgeführt und zum Erwärmen der Edukte oder bei der Destillation der Produkte eingesetzt werden.

Die in den genannten Dokumenten beschriebene Kopplung der Abfuhr der Reaktionswärme mit der Gewinnung von Wertprodukten ist ebenfalls mit Nachteilen verbunden. Eine gegebenenfalls notwendige Wärmeisolierung von dem Hydroformylierungsreaktor nachgeschalteten Apparaturen, wie Phasentrenngefäßen, ist aufwendig und kostspielig. Zudem kann man die Kopplung von Wärme- und Stofftransport nur begrenzt an die Reaktionsbedingungen anpassen, wenn beispielsweise eine stärkere Wärmeabfuhr erforderlich ist, als mit der Gewinnung von Produkten vereinbar ist.

Die Patentanmeldung DE-A-19836807 beschreibt ein Hydroformylierungverfahren, das eine flüssige Phase enthält. Dieser Phase wird ein Teilstrom entommen und über einen Wärmetauscher geleitet. Anschließend wird der Strom ohne eine Abtrennung einer stofflichen Komponente in die Reaktionszone zurückgeleitet.

Der vorliegenden Erfindung liegt die Aufgabe zu Grunde, ein Verfahren zur Hydroformylierung zur Verfügung zu stellen, bei dem man die Reaktionswärme effektiv und einfach abführen und möglichst wieder nutzbringend einsetzen kann.

Diese Aufgabe wird erfindungsgemäß durch ein Verfahren zur Hydroformylierung gelöst, bei dem man wenigstens ein Olefin mit 2 bis 6 Kohlenstoffatomen mit Kohlenmonoxid und Wasserstoff in Gegenwart eines Hydroformylierungskatalysators in einer Reaktionszone kontinuierlich umsetzt, die eine flüssige Phase enthält und aus der flüssigen Phase einen Strom S) entnimmt, diesem Strom Wärme entzieht und ihn anschließend ohne Abtrennung einer stofflichen Komponente in die Reaktionszone zurückführt wobei die Umsetzung in dem Strom S) zumindest bis zum Entzug der Wärme fortschreitet und man in diesen Strom Kohlenmonoxid und/oder Wasserstoff einspeist, bevor er an ihnen soweit verarmt ist, dass die verbleibenden Komponenten unerwünschte Nebenreaktionen eingehen und/oder die Hydroformylierung im Wesentlichen zum Erliegen kommt.

Nach dem erfindungsgemäßen Verfahren erfolgt die Umsetzung vorzugsweise in einer Reaktionsapparatur, die eine Flüssigphase und eine Gasphase enthält. Die Flüssigphase ist vorzugsweise im Wesentlichen homogen und enthält neben dem Hydroformylierungskatalysator die gelösten Reaktanten. Zur Abführung der Reaktionswärme wird der Flüssigphase ein Strom S) entnommen, diesem Wärme durch Kühlung entzogen und anschließend in die Reaktionszone zurückgeführt. Dabei erfolgt nach dem erfindungsgemäßen Verfahren keine Abtrennung einer stofflichen Komponente, wie z. B. von Produkten, Edukten etc., aus dem der Reaktionszone zum Entzug der Wärme entnommenen Strom.

Vorzugsweise wird dem Strom die Wärme durch Inkontaktbringen mit einem Wärmeaustauscher entzogen. Geeignete Wärmeaustauscher sind die üblichen zur Wärmeübertragung bestimmten Apparaturen, in welchen Wärme von einem Ort oder Medium zu einem anderen transportiert wird. Sie können in üblichen Bauarten ausgeführt sein, wie z. B. Platten-, Ringnut-, Rippenrohr-, Lamellen-, Rohrbündel-, Spaltrohr-, Teller-, Spiral-, Block-, Kratz-, Schnecken-, Wendel-, Wirbelschicht-, Kerzen-, gekühlte Umwälz- sowie Zwei- und Dreirohrschlangenaustauscher und Rekuperatoren. Der Entzug der Wärme kann z. B. durch Inkontaktbringen des Stroms S) mit einem Kühlmedium erfolgen. Dazu können die Wärmeaustauscher beispielsweise als Wasserkühler oder Luftkühler ausgestaltet sein. Nach einer bevorzugten Ausführungsform wird die dem Strom S) entzogene Wärme jedoch in einem wärmeverbrauchenden Schritt des Hydroformylierungsverfahrens oder eines anderen Verfahrens eingesetzt. Dazu ist es von Vorteil, den der Hydroformylierungsreaktion entnommenen Strom S) an den Ort des Wärmeverbrauchs zu führen, ohne die enthaltene Wärme zwischenzeitlich auf ein Hilfsmedium zu übertra-gen. Dies kann z. B. in üblichen, gegebenenfalls wärmeisolierten und druckfest ausgerüsteten Rohrleitungen erfolgen. Geeignete wärmeverbrauchende Schritte des Hydroformylierungsverfahrens, die sich zum Einsatz der gewonnenen Prozessabwärme eignen, werden im Folgenden näher beschrieben. Dazu zählen insbesondere:
- die Erwärmung der Reaktanten (Synthesegas und/oder Einsatzolefin),
- thermische Trenn- bzw. Reinigungsverfahren bei der Aufarbeitung des Reaktionsaustrags.

Im Allgemeinen weist der in die Reaktionszone zurückgeführte Strom eine Temperatur auf, die 5 bis 15 °C unterhalb der Temperatur in der Reaktionszone liegt.

Geeignete Olefine, die nach dem erfindungsgemäßen Verfahren hydroformyliert werden können, enthalten 2 bis 6 Kohlenstoffatome. Es kann sich um geradkettige, verzweigte oder cyclische Olefine handeln. Bevorzugte Beispiele geeigneter Olefine sind Ethen, Propen, 1-Buten und 2-Buten. Das Olefin wird im Allgemeinen in Form eines olefinhaltigen Zulaufs in die Reaktionszone eingespeist. Nach einer geeigneten Ausführungsform des erfindungsgemäßen Verfahrens kann der olefinhaltige Zulauf und/oder das Synthesegas vor dem Einspeisen in die Reaktionszone unter Einsatz von der Reaktionszone entzogenen Wärme erwärmt werden. Der olefinhaltige Zulauf kann ein einzelnes Olefin oder ein Gemisch von Olefinen enthalten. Das erfindungsgemäße Verfahren ist besonders geeignet für den Einsatz von Propylen unter Erzeugung von n- und iso-Butanal. Mit Vorteil ist jedoch auch der Einsatz von Ethylen unter Erzeugung von Propionaldehyd bzw. von 1-Buten unter Erzeugung von n- und iso-Valeraldehyd möglich. Der eingesetzte olefinhaltige Zulauf kann eine Beimischung eines gesättigten Kohlenwasserstoffs, in der Regel eines gesättigten Kohlenwasserstoffs gleicher Kohlenstoffzahl wie das eingesetzte Olefin, enthalten. Um ein kontinuierliches Ansteigen der Konzentration des gesättigten Kohlenwasserstoffs in der Reaktionszone zu verhindern, ist es dann in der Regel erforderlich, an einer Stelle des Verfahrens eine Abtrennung des gesättigten Kohlenwasserstoffs vorzusehen.

Kohlenmonoxid und Wasserstoff werden üblicherweise in Form eines Gemischs, dem sogenannten Synthesegas, eingesetzt. Die Zusammensetzung des im erfindungsgemäßen Verfahren eingesetzten Synthesegases kann in weiten Bereichen variieren. Das molare Verhältnis von Kohlenmonoxid und Wasserstoff beträgt in der Regel 2:1 bis 1:2, insbesondere etwa 45:55 bis 50:50.

Die Temperatur bei der Hydroformylierungsreaktion liegt im Allgemeinen in einem Bereich von 50 bis 200 °C, vorzugsweise 60 bis 190 °C, insbesondere 90 bis 190 °C. Die Umsetzung wird vorzugsweise bei einem Druck im Bereich von 10 bis 700 bar, vorzugsweise 15 bis 200 bar, insbesondere 15 bis 60 bar durchgeführt. Der Reaktionsdruck kann in Abhängigkeit von der Aktivität des eingesetzten Hydroformylierungskatalysators variiert werden.

Geeignete druckfeste Reaktionsapparaturen für die Hydroformylierung sind dem Fachmann bekannt. Dazu zählen die allgemein üblichen Reaktoren für Gas-flüssig-Reaktionen, wie z. B. Gasumlaufreaktoren oder Blasensäulen, die gegebenenfalls durch Einbauten unterteilt sein können. Vorteilhafterweise ermöglicht das erfindungsgemäße Verfahren den Einsatz einfacher und kostengünstiger Reaktoren, bei denen auf den Einsatz aufwendiger Kühlvorrichtungen, wie an der Außenfläche oder im Inneren angebrachten Kühlschlangen oder Kühlregistern, verzichtet werden kann. Bevorzugt sind Blasensäulenreaktoren. Vorteilhafterweise wird die Reaktorgröße bei dem erfindungsgemäßen Verfahren praktisch nicht, wie bei aus dem Stand der Technik bekannten Verfahren, durch die Notwendigkeit zur Kühlung des Reaktionsansatzes limitiert.

Geeignete Hydroformylierungskatalysatoren sind die üblichen, dem Fachmann bekannten Übergangsmetallverbindungen und -komplexe, die sowohl mit als auch ohne Cokatalysatoren eingesetzt werden können. Bevorzugt handelt es sich bei dem Übergangsmetall um ein Metall der VIII. Nebengruppe des Periodensystems und insbesondere um Co, Ru, Rh, Pd, Pt, Os oder Ir, speziell um Rh, Co, Ir oder Ru.

Geeignete Komplexverbindungen sind beispielsweise die Carbonylverbindungen der genannten Metalle sowie Komplexe, deren Liganden ausgewählt sind unter Aminen, Arylphosphinen, Alkylphosphinen, Arylalkylphosphinen, Olefinen oder Dienen und Mischungen davon.

Es eignen sich z. B. Rhodiumkomplexe der allgemeinen Formel RhXₘL¹L²(L³)ₙ, worin
- X: für Halogenid, vorzugsweise Chlorid oder Bromid, Alkyl- oder Arylcarboxylat, Acetylacetonat, Aryl- oder Alkylsulfonat, insbesondere Phenylsulfonat und Toluolsulfonat, Hydrid oder das Diphenyltriazin-Anion,
- L¹, L², L³: unabhängig voneinander für CO, Olefine, Cycloolefine, vorzugsweise Cyclooctadien (COD), Dibenzophosphol, Benzonitril, PR₃ oder R₂P-A-PR₂, m für 1 oder 3 und n für 0, 1 oder 2 stehen. Unter R (die Reste R können gleich oder verschieden sein) sind Alkyl-, Cycloalkyl- und Arylreste zu verstehen, vorzugsweise Phenyl, p-Tolyl, m-Tolyl, p-Ethylphenyl, p-Cumyl, p-t-Butylphenyl, P-C₁-C₄-Alkoxyphenyl, vorzugsweise p-Anisyl, Xylyl, Mesityl, p-Hydroxyphenyl, das gegebenenfalls auch ethoxyliert vorliegen kann, Sulfophenyl, Isopropyl, C₁-C₄-Alkoxy, Cyclopentyl oder Cyclohexyl. A steht für 1,2-Ethylen oder 1,3-Propylen. Bevorzugt stehen L¹, L² oder L³ unabhängig voneinander für CO, COD, P(Phenyl)₃, P(i-Propyl)₃, P(Anisyl)₃, P(OC₂H₅)₃, P(Cyclohexyl)₃, Dibenzophosphol oder Benzonitril.
- X: steht bevorzugt für Hydrid, Chlorid, Bromid, Acetat, Tosylat, Acetylacetonat oder das Diphenyltriazin-Anion, insbesondere für Hydrid, Chlorid oder Acetat.

Bevorzugte Hydroformylierungskatalysatoren sind phosphorhaltige Rhodiumkatalysatoren, wie sie z. B. unter Hydroformylierungsbedingungen in situ aus einer Rhodiumquelle und einem Triarylphosphin, z. B. Triphenylphosphin, gebildet werden, wie RhH(CO)₂(PPh₃)₂ oder RhH(CO)(PPh₃)₃.

Geeignete Hydroformylierungskatalysatoren werden z. B. in Beller et al., Journal of Molecular Catalysis A, 104 (1995), S. 17-85, beschrieben, worauf hier in vollem Umfang Bezug genommen wird.

Eine bevorzugte Ausführungsform ist ein kontinuierliches Verfahren, bei dem man
i) in eine Reaktionszone einen olefinhaltigen Zulauf, der wenigstens ein Olefin mit 2 bis 6 Kohlenstoffatomen enthält, sowie Kohlenmonoxid und Wasserstoff einspeist und in Gegenwart eines Hydroformylierungskatalysators umsetzt, wobei die Reaktionszone eine flüssige Phase enthält, aus der man einen Strom S) entnimmt, diesem Strom Wärme entzieht und ihn anschließend ohne Abtrennung eine stofflichen Komponente in die Reaktionszone zurückführt, wobei die Umsetzung in dem Strom S) zumindest bis zum Entzug der Wärme fortschreitet und man in diesen Strom Kohlenmonoxid und/oder Wasserstoff einspeist, bevor er an ihnen soweit verarmt ist, dass die verbleibenden Komponenten unerwünschte Nebenreaktionen eingehen und/oder die Hydroformylierung im Wesentlichen zum Erliegen kommt.
ii) der Reaktionszone einen Austrag entnimmt, der einer ein- oder mehrstufigen Trennoperation unter Gewinnung zumindest eines die Hauptmenge des Hydroformylierungsprodukts enthaltenden Stroms und eines die Hauptmenge des nicht umgesetzten Olefins enthaltenden Stroms unterzogen wird, und
iii) den die Hauptmenge des nicht umgesetzten Olefins enthaltenden Stroms zumindest teilweise in die Reaktionszone zurückführt.

In der Reaktionszone findet im Allgemeinen, bezogen auf das eingespeiste Olefin, pro Durchgang ein Teilumsatz statt. Der Umsatz beträgt im Allgemeinen 10 bis 90 % bezogen auf das eingespeiste Olefin.

Der Austrag aus der Reaktionszone kann einer ein- oder mehrstufigen Trennoperation unterzogen werden, wobei zumindest ein die Hauptmenge des Hydroformylierungsproduktes enthaltender Strom und ein die Hauptmenge des nicht umgesetzten Olefins enthaltender Strom erhalten wird. Die in dem eingesetzten Olefin gegebenenfalls enthaltenen sowie die gegebenenfalls als Nebenprodukt der Hydroformylierung gebildeten gesättigten Kohlenwasserstoffe sind im Allgemeinen ebenfalls in dem die Hauptmenge des Olefins enthaltenden Strom enthalten. Je nach Austragsverfahren werden gegebenenfalls weitere Ströme erhalten, wie synthesegashaltige Abgase, hochsiedende Nebenprodukte der Hydroformylierung und/oder Hydroformylierungskatalysator enthaltende Ströme, die - gegebenenfalls nach Aufarbeitung - ganz oder teilweise in die Reaktionszone zurückgeführt oder aus dem Verfahren ausgeschleust werden. Vom Austrag aus der Reaktionszone können beispielsweise zuerst das Hydroformylierungsprodukt und gegebenenfalls höher als das Hydroformylierungsprodukt siedende Anteile abgetrennt werden. Anschließend kann nicht umgesetztes Olefin, gegebenenfalls im Gemisch mit gesättigtem Kohlenwasserstoff, auskondensiert werden. Der bei den einzelnen Trennschritten anfallende Wärmebedarf kann, zumindest teilweise, durch die der Reaktionszone entzogene Wärme gedeckt werden.

Nach einer geeigneten Ausführungsform erhält man den im Wesentlichen aus nicht umgesetztem Olefin und gegebenenfalls gesättigtem Kohlenwasserstoff bestehenden Strom jedoch dadurch, dass man vom Austrag aus der Reaktionszone zuerst ein rohes Hydroformylierungsprodukt abtrennt, welches nicht umgesetztes Olefin und gegebenenfalls gesättigten Kohlenwasserstoff gelöst enthält, und das flüssige rohe Hydroformylierungsprodukt dann einem Entgasungsschritt unterzieht, bei dem ein Strom anfällt, der im Wesentlichen aus nicht umgesetztem Olefin und gegebenenfalls gesättigtem Kohlenwasserstoff besteht. Der vom rohen Hydroformylierungsprodukt befreite Reaktionsaustrag wird in der Regel ganz oder teilweise in die Reaktionszone zurückgeführt. Zur Entgasung kann das rohe Hydroformylierungsprodukt entspannt, erwärmt und/oder mit einem Strippgas, wie Synthesegas oder Stickstoff, behandelt werden. Zweckmäßigerweise führt man die Entgasung in einer Kolonne durch, wobei z. B. das rohe Hydroformylierungsprodukt im Bereich der Kolonnenmitte eingespeist wird, am Kolonnensumpf das entgaste Hydroformylierungsprodukt abgezogen wird, das der weiteren Aufarbeitung zugeführt wird, und am Kolonnenkopf ein flüssiger oder gasförmiger Strom abgezogen wird, der im Wesentlichen aus nicht umgesetztem Olefin und gesättigtem Kohlenwasserstoff besteht. Bevorzugt wird der Wärmebedarf der Entgasungskolonne, zumindest teilweise, durch die der Reaktionszone entzogene Wärme gedeckt.

Die Abtrennung des rohen Hydroformylierungsproduktes vom Austrag aus der Reaktionszone kann auf verschiedene Weise erfolgen. Es kann zum einen das sogenannte Flüssigaustragsverfahren zum Einsatz kommen, bei dem der im Wesentlichen, bis auf das im Überschuss zur Hydroformylierung eingesetzte Synthesegas, flüssige Austrag aus der Reaktionszone entspannt wird, wobei in Folge der Druckerniedrigung der Austrag in eine Flüssigphase, die im Wesentlichen aus hochsiedenden Nebenprodukten, dem homogen gelösten Hydroformylierungskatalysator, gegebenenfalls einem Teil des Hydroformylierungsprodukts und geringen Mengen an nicht umgesetztem Olefin und an gesättigtem Kohlenwasserstoff besteht, und eine Gasphase, die im Wesentlichen aus Hydroformylierungsprodukt, nicht umgesetztem Olefin und gesättigtem Kohlenwasserstoff sowie nicht umgesetztem Synthesegas besteht, aufgetrennt wird. Die Flüssigphase kann als Rückführstrom wieder in den Reaktor geleitet werden. Durch zumindest partielle Kondensation der Gasphase wird das rohe Hydroformylierungsprodukt erhalten. Die bei der Kondensation zurückbleibende Gasphase wird ganz oder teilweise in die Reaktionszone zurückgeführt.

Mit Vorteil kann die in der Entspannungsstufe primär anfallende Gas- und Flüssigphase nach dem in der WO 97/07086 beschriebenen Verfahren aufgearbeitet werden. Zu diesem Zweck wird die Flüssigphase erwärmt und in den oberen Bereich einer Kolonne eingeleitet, während die Gasphase in den Sumpf der Kolonne eingeleitet wird. Zum Erwärmen der Flüssigphase kann, zumindest teilweise, die der Reaktionszone entzogene Wärme eingesetzt werden. Flüssigphase und Gasphase werden im Gegenstrom geführt. Um den gegenseitigen Kontakt der Phasen zu erhöhen, ist die Kolonne vorzugsweise mit Füllkörpern gefüllt. Durch den innigen Kontakt der Gasphase mit der Flüssigphase werden die in der Flüssigphase vorhandenen Restmengen an Hydroformylierungsprodukt, nicht umgesetztem Olefin und gesättigtem Kohlenwasserstoff in die Gasphase transferiert, so dass der die Kolonne am Kopf verlassende Gasstrom im Vergleich zu dem am unteren Ende der Kolonne eingeführten Gasstrom an Hydroformylierungsprodukt, nicht umgesetztem Olefin und gesättigtem Kohlenwasserstoff angereichert ist. Die weitere Aufarbeitung des die Kolonne verlassenden Gasstroms und der die Kolonne verlassenden Flüssigphase erfolgt in üblicher Weise, beispielsweise wie oben beschrieben.

Alternativ kann man insbesondere bei Einsatz von C₂-C₄-Olefinen nach dem sogenannten Kreisgasverfahren (gas recycle) arbeiten, bei dem aus dem Gasraum des Hydroformylierungsreaktors ein Gasstrom abgezogen wird. Dieser Gasstrom besteht im Wesentlichen aus Synthesegas, nicht umgesetztem Olefin und gegebenenfalls gesättigtem Kohlenwasserstoff, wobei nach Maßgabe des Dampfdrucks im Hydroformylierungsreaktor das bei der Hydroformylierungsreaktion gebildete Hydroformylierungsprodukt mitgeführt wird. Aus dem Gasstrom wird das mitgeführte Hydroformylierungsprodukt z. B. durch Abkühlen auskondensiert, und der vom Flüssiganteil befreite Gasstrom wird zurück in den Hydroformylierungsreaktor geführt.

Der im Wesentlichen aus nicht umgesetztem Olefin und gegebenenfalls gesättigtem Kohlenwasserstoff bestehende Strom enthält z. B. 50 bis 100 Gew.-%, vorzugsweise 50 bis 95 Gew.-%, Olefin und gegebenenfalls 5 bis 50 Gew.-% gesättigte Kohlenwasserstoffe.

Sofern der die Hauptmenge des nicht umgesetzten Olefins enthaltende Strom noch gesättigte Kohlenwasserstoffe aufweist, kann er durch übliche Trennverfahren, wie Destillation oder Membranfiltration, in eine Olefin-angereicherte Fraktion und eine Olefin-abgereicherte Fraktion getrennt werden. Die Destillation (Rektifikation) erfolgt üblicherweise bei tiefer Temperatur und/oder erhöhtem Druck, wobei die genauen Temperatur- und/oder Druckbedingungen von Faktoren, wie der Kohlenstoffanzahl des zu trennenden Olefins/gesättigten Kohlenwasserstoffs, usw. abhängen. Die Rektifikation erfolgt im Allgemeinen in einer Kolonne, die mit einer ausreichend großen Zahl von Rektifikationsböden versehen ist. Kolonnen für derartige Trennaufgaben sind an sich bekannt und werden z. B. zur Trennung von Olefinen und gesättigten Kohlenwasserstoffen eingesetzt, die im Spaltgas eines Steamcrackers enthalten sind. Der aufzutrennende Strom kann wahlweise gasförmig oder flüssig in die Kolonne eingeführt werden, vorzugsweise im Bereich der Kolonnenmitte. Am Kopf oder oberen Bereich der Kolonne kann zweckmäßigerweise die Olefin-angereicherte Fraktion, am Sumpf oder unteren Bereich der Kolonne die Olefin-abgereicherte Fraktion abgenommen werden. Der Wärmebedarf der Trennung in eine Olefin-angereicherte und eine Olefin-abgereicherte Fraktion kann, zumindest teilweise, durch die der Reaktionszone entzogene Wärme gedeckt werden.

In der Regel strebt man an, als Olefin-abgereicherte Fraktion möglichst reinen gesättigten Kohlenwasserstoff zu gewinnen, der ohne größeren Verlust an Olefin aus dem Verfahren ausgeleitet werden kann. Demgegenüber strebt man bei der Olefin-angereicherten Fraktion im Allgemeinen nicht reines Olefin an, sondern lässt darin einen gewissen Gehalt an gesättigten Kohlenwasserstoffen zu, um den Aufwand der Trennung geringer zu halten.

Nach einer bevorzugten Ausführungsform wird im erfindungsgemäßen Verfahren ein propylenhaltiger Zulauf eingesetzt. Enthält dieser eine Beimischung von Propan oder wird bei der Hydroformylierung als Nebenprodukt Propan gebildet, so fällt als zu trennender Strom ein Gemisch von Propylen und Propan an. Die Auftrennung dieses Stroms in eine Propylen-angereicherte Fraktion und eine Propylen-abgereicherte Fraktion gelingt in einer geeigneten Destillationskolonne unter Druck, einem sogenannten C₃-Splitter. Die Kolonne wird vorzugsweise so gefahren, dass am Kopf eine Propylen-angereicherte Fraktion anfällt, die direkt in die Reaktionszone zurückgeführt werden kann, und am Sumpf weitgehend reines Propan abgezogen werden kann, das ohne Verlust an Propylen aus dem System entfernt werden kann. Typische Betriebsbedingungen des C₃-Splitters sind: 20 bis 25 bar Kopfdruck, 60 bis 70°C Sumpftemperatur, 100 bis 150 theoretische Böden. Der Wärmebedarf des C₃-Splitters wird vorzugsweise, zumindest teilweise, durch die der Reaktionszone entzogene Wärme gedeckt.

Die olefin-abgereicherte Fraktion wird aus dem System ausgeleitet. Sie kann z. B. verbrannt werden. Sie kann ferner als Einsatzstoff für chemische Umsetzungen, z. B. im Steamcracker, dienen.

Der nach dem erfindungsgemäßen Verfahren aus der flüssigen Phase zum Entzug der Wärme entnommene Strom S) enthält alle Einsatzstoffe der Hydroformylierungsreaktion sowie den Katalysator und befindet sich, zumindest bis zum Inkontaktbringen mit dem Wärmeaustauscher, auf Reaktionstemperatur. Somit läuft die Hydroformylierungsreaktion in dem Strom zumindest so lange weiter, bis durch den Wärmeentzug die Reaktionsgeschwindigkeit deutlich herabgesetzt wird, oder bis der Strom durch das Fortschreiten der Reaktion an einem Produkt soweit verarmt, dass die Hydroformylierung zum Stillstand kommt. Diese Verarmung betrifft in der Regel die in dem Strom gelösten gasförmigen Komponenten und dabei speziell das im Allgemeinen in geringster Konzentration, bezogen auf die Edukte, vorhandene Kohlenmonoxid. Bei einer Verarmung des Stroms an Kohlenmonoxid kann dann als unerwünschte Nebenreaktion die Hydrierung des Olefins durch noch vorhandenen Wasserstoff auftreten. Diese Bildung gesättigter Kohlenwasserstoffe bedeutet einen unerwünschten Verlust an teuren Einsatzstoffen. Um diesen Nachteil zu vermeiden und das bis zum Inkontaktbringen des Stroms mit dem Wärmeaustauscher, oder auch darüber hinaus, zur Verfügung stehende Rohrvolumen in vorteilhafter Weise als Reaktorraum zu nutzen, wird in den Strom die Reaktionskomponente, an der er zu verarmen droht, d.h. Kohlenmonoxid /oder Wasserstoff eingespeist. Die Einspeisung von Komponenten in den zum Entzug der Wärme aus der Reaktionszone ausgeschleusten Strom erfolgt nach üblichen, dem Fachmann bekannten Verfahren, z. B. durch einfaches Zusammenführen der Ströme an einer oder mehreren verschiedenen Einspeisungsstellen, oder über Gasverteiler, Fritten, Lochplatten etc.

Besonders bevorzugt ist ein Verfahren, bei dem man in den Strom Kohlenmonoxid einspeist, bevor er daran soweit verarmt ist, dass das enthaltene Olefin mit noch vorhandenem Wasserstoff unter Bildung von Hydrierungsprodukten reagiert. Dies ist insbesondere vorteilhaft beim Einsatz von Propen als Olefin, da die Propen/Propan-Trennung, wie zuvor beschrieben, apparativ äußerst aufwendig ist. Vorteilhafterweise kann bei der beschriebenen Vorgehensweise die Bildung von Propan in dem Kühlkreislauf vollständig oder weitgehend unterbunden werden.

Die Erfindung wird anhand der folgenden, nicht einschränkenden Beispiele näher erläutert.

### Beispiele

### Vergleichsbeispiel 1 (Hydroformylierung von Propen):

Zur Hydroformylierung wird ein Reaktor eingesetzt, der zur Abtrennung und Ausschleusung von Propan mit einer Entgasungskolonne und einem C₃-Splitter verbunden ist. Die Kühlung des Reaktorinhalts erfolgt über einen Ausschleusstrom in einen äußeren Kühlkreis, über den Reaktionswärme der Hydroformylierung abgeführt wird. Dem Reaktor werden kontinuierlich 10 t/h chemical grade-Propylen (Propangehalt 5 %) und etwa 6,5 t/h Oxogas (CO-Gehalt in der Gasphase des Hydroformylierungsreaktors = 4 %) als Einsatzstoffe zugeführt.

Die Menge des ausgeschleusten Propanstroms beträgt 1 t/h, wobei 0,5 t/h auf mit dem eingesetzten Propylen zugeführtes Propan und weitere 0,5 t/h auf durch Hydrierung von Propen gebildetes Propan entfallen. Der Dampfverbrauch der Entgasungskolonne beträgt 1,8 t/h, der des C₃-Splitters 3,4 t/h.

### Beispiel 2: Vergleichsbeispiel

In einem Reaktorsystem gemäß Beispiel 1 wird die Reaktionswärme der Hydroformylierung als Energiequelle für die Entgasungskolonne und den C₃-Splitter genutzt, indem der äußere Kühlkreislauf durch die entsprechenden Verdampfer der Kolonnen geführt wird. Der Dampfverbrauch des Splitters entfällt dabei ganz, der der Entgasungskolonne sinkt auf 0,4 t/h. Die Einsparung an Dampf beträgt damit insgesamt 4,8 t/h.

### Beispiel 3:

Es wird gemäß Beispiel 2 verfahren, wobei dem Wärmekreislauf ein Strom vom 220 kg CO/h zudosiert wird. Dadurch sinkt der Anteil des durch unerwünschte Hydrierung von Propen im äußeren Kühlkreislauf gebildeten Propans, und der ausgeschleuste Propanstrom verringert sich auf 900 kg/h.

### Beispiel 4:

Es wird wie in Beispiel 2 verfahren, wobei dem Wärmekreislauf ein Strom von 250 kg/h Oxogas zudosiert wird. Der ausgeschleuste Propanstrom sinkt dadurch ebenfalls auf 900 kg/h.

## Patentansprüche

1. Verfahren zur Hydroformylierung, bei dem man wenigstens ein Olefin mit 2 bis 6 Kohlenstoffatomen mit Kohlenmonoxid und Wasserstoff in Gegenwart eines Hydroformylierungskatalysators in einer Reaktionszone kontinuierlich umsetzt, die eine flüssige Phase enthält und aus der flüssigen Phase einen Strom S) entnimmt, diesem Strom Wärme entzieht und ihn anschließend ohne Abtrennung einer stofflichen Komponente in die Reaktionszone zurückführt, wobei die Umsetzung in dem Strom S) zumindest bis zum Entzug der Wärme fortschreitet und man in diesen Strom Kohlenmonoxid und/oder Wasserstoff einspeist, bevor er an ihnen soweit verarmt ist, dass die verbleibenden Komponenten unerwünschte Nebenreaktionen eingehen und/oder die Hydroformylierung im wesentlichen zum Erliegen kommt.

2. Verfahren nach Anspruch 1, bei dem man dem Strom S) die wärme durch Inkontaktbringen mit einem Wärmeaustauscher entzieht.

3. Verfahren nach einem der vorhergehenden Ansprüche, bei dem man die dem Strom S) entzogene wärme in einem wärmeverbrauchenden Schritt des Hydroformylierungsverfahrens oder eines anderen Verfahrens einsetzt.

4. Verfahren nach einem der vorhergehenden Ansprüche, bei dem man dem Strom S) die Wärme ohne Einsatz eines Hilfsmediums zur Wärmeübertragung entzieht.

5. Verfahren nach einem der vorhergehenden Ansprüche, bei dem man
i) in eine Reaktionszone einen olefinhaltigen Zulauf, der wenigstens ein Olefin mit 2 bis 6 Kohlenstoffatomen enthält, sowie Kohlenmonoxid und Wasserstoff einspeist und in Gegenwart eines Hydroformylierungskatalysators umsetzt, wobei die Reaktionszone eine flüssige Phase enthält, aus der man einen Strom S) entnimmt, diesem Strom wärme entzieht und ihn anschließend ohne Abtrennung einer stofflichen Komponente in die Reaktionszone zurückführt,
ii) der Reaktionszone einen Austrag entnimmt, der einer ein- oder mehrstufigen Trennoperation unter Gewinnung zumindest eines die Hauptmenge des Hydroformylierungsprodukts enthaltenden Stroms und eines die Hauptmenge des nicht umgesetzten olefins enthaltenden Stroms unterzogen wird, und
iii) den die Hauptmenge des nicht umgesetzten olefins enthaltenden Stroms zumindest teilweise in die Reaktionszone zurückführt.

6. Verfahren nach Anspruch 5, bei dem man den die Hauptmenge des nicht umgesetzten olefins enthaltenden Strom **dadurch** erhält, dass man vom Austrag aus der Reaktions zone zuerst ein rohes Hydroformylierungsprodukt abtrennt und einem Entgasungsschritt unterzieht, wobei zur Deckung des Wärmebedarfs des Entgasungsschritts, zumindest teilweise, die dem Strom S) entzogene Wärme eingesetzt wird.

7. Verfahren nach einem der Ansprüche 5 oder 6, bei dem der die Hauptmenge des nicht umgesetzten Olefins enthaltende Strom zusätzlich gesättigte Kohlenwasserstoffe aufweist und er durch Destillation in eine Olefin-angereicherte Fraktion und eine olefin-abgereicherte Fraktion getrennt wird, wobei zur Deckung des wärmebedarfs der Destillation, zumindest teilweise, die dem Strom S) entzogene Wärme eingesetzt wird.

8. Verfahren nach einem der vorhergehenden Ansprüche, bei dem man in den Strom S) Kohlenmonoxid einspeist, bevor er daran soweit verarmt ist, dass das olefin mit dem Wasserstoff unter Bildung von Hydrierungsprodukten reagiert.

## Claims

1. A hydroformylation process in which at least one olefin having from 2 to 6 carbon atoms is reacted continuously with carbon monoxide and hydrogen in the presence of a hydroformylation catalyst in a reaction zone in which a liquid phase is present and a stream S) is taken from the liquid phase, heat is removed from this stream and the stream is subsequently returned to the reaction zone without removal of a material component, wherein the reaction proceeds in the stream S) at least until the removal of the heat and carbon monoxide and/or hydrogen are/is fed into this stream before it is depleted in these/this component(s) to such an extent that the remaining components undergo undesirable secondary reactions and/or the hydroformylation essentially stops.

2. The process according to claim 1, wherein the heat is withdrawn from the stream S) by bringing it into contact with a heat exchanger.

3. The process according to either of the preceding claims, wherein the heat withdrawn from the stream S) is used in a heat-consuming step of the hydroformylation process or of another process.

4. The process according to any of the preceding claims, wherein the heat is withdrawn from the stream S) without use of an auxiliary medium for heat transfer.

5. The process according to any of the preceding claims, wherein
i) an olefin-containing feed stream comprising at least one olefin having from 2 to 6 carbon atoms and also carbon monoxide and hydrogen are fed into a reaction zone and reacted in the presence of a hydroformylation catalyst, where a liquid phase is present in the reaction zone and a stream S) is taken from this liquid phase, heat is withdrawn from this stream and the stream is subsequently returned to the reaction zone without removal of a material component,
ii) an output is taken from the reaction zone and is subjected to a single-stage or multistage separation operation to give at least one stream comprising the major part of the hydroformylation product and a stream comprising the major part of the unreacted olefin, and
iii)at least part of the stream comprising the major part of the unreacted olefin is returned to the reaction zone.

6. The process according to claim 5, wherein the stream comprising the major part of the unreacted olefin is obtained by firstly separating off a crude hydroformylation product from the output from the reaction zone and subjecting it to a degassing step, with the heat withdrawn from the stream S) being used to cover at least part of the heat requirement of the degassing step.

7. The process according to claim 5 or 6, wherein the stream comprising the major part of the unreacted olefin further comprises saturated hydrocarbons and is separated by distillation into an olefin-enriched fraction and an olefin-depleted fraction, with the heat withdrawn from the stream S) being used to cover at least part of the heat requirement of the distillation.

8. The process according to any one of the preceding claims, wherein carbon monoxide is fed into the stream S) before it is depleted in this to such an extent that the olefin reacts with the hydrogen to form hydrogenation products.

## Revendications

1. Procédé d'hydroformylation, dans lequel on fait réagir en continu au moins une oléfine comportant 2 à 6 atomes de carbone avec du monoxyde de carbone et de l'hydrogène, en présence d'un catalyseur d'hydroformylation, dans une zone réactionnelle qui contient une phase liquide, et on prélève de la phase liquide un courant S), on soutire de la chaleur de ce courant et on le recycle ensuite dans la zone réactionnelle sans séparation d'un composant matériel, la réaction se poursuivant dans le courant S) au moins jusqu'au soutirage de la chaleur, et on alimente dans ce courant du monoxyde de carbone et/ou de l'hydrogène avant qu'il ne soit appauvri en ceux-ci à tel point que les composants subsistants donnent lieu à des réactions secondaires non souhaitées et/ou que l'hydroformylation vient sensiblement à cesser.

2. Procédé suivant la revendication 1, dans lequel on soutire la chaleur du courant S) par mise en contact avec un échangeur de chaleur.

3. Procédé suivant l'une des revendications précédentes, dans lequel on met en oeuvre la chaleur soutirée du courant S) dans une étape consommant de la chaleur du procédé d'hydroformylation ou d'un autre procédé.

4. Procédé suivant l'une des revendications précédentes, dans lequel on soutire la chaleur du courant S) sans mise en oeuvre d'un milieu auxiliaire pour le transfert thermique.

5. Procédé suivant l'une des revendications précédentes, dans lequel
i) on amène dans une zone réactionnelle une alimentation contenant de l'oléfine, qui contient au moins une oléfine comportant 2 à 6 atomes de carbone, ainsi que du monoxyde de carbone et de l'hydrogène, et on fait réagir en présence d'un catalyseur d'hydroformylation, la zone réactionnelle contenant une phase liquide à partir de laquelle on prélève un courant S), on soutire de ce courant de la chaleur et on le recycle ensuite dans la zone réactionnelle sans séparation d'un composant matériel,
ii) on prélève de la zone réactionnelle un effluent qui est soumis à une opération de séparation en une ou plusieurs étapes avec production d'au moins un courant contenant la quantité principale du produit d'hydroformylation et d'un courant contenant la quantité principale de l'oléfine n'ayant pas réagi, et
iii) on recycle au moins partiellement dans la zone réactionnelle le courant contenant la quantité principale de l'oléfine n'ayant pas réagi.

6. Procédé suivant la revendication 5, dans lequel on obtient le courant contenant la quantité principale de l'oléfine n'ayant pas réagi par le fait qu'on isole de l'effluent de la zone réactionnelle tout d'abord un produit d'hydroformylation brut et on le soumet à une étape de dégazage, la chaleur soutirée du courant S) étant, au moins partiellement, mise en oeuvre pour couvrir le besoin de chaleur de l'étape de dégazage.

7. Procédé suivant l'une des revendications 5 ou 6, dans lequel le courant contenant la quantité principale de l'oléfine n'ayant pas réagi présente en supplément des hydrocarbures saturés et il est séparé par distillation en une fraction enrichie en oléfine et en une fraction appauvrie en oléfine, la chaleur soutirée du courant S) étant, au moins partiellement, mise en oeuvre pour couvrir le besoin de chaleur de la distillation.

8. Procédé suivant l'une des revendications précédentes, dans lequel on alimente du monoxyde de carbone dans le courant S) avant qu'il ne soit appauvri en celui-ci à tel point que l'oléfine réagit avec l'hydrogène avec formation de produits d'hydrogénation.
